# EUROPEAN PATENT SPECIFICATION

(11) **EP 3 156 550 B1**
(45) Date of publication and mention of the grant of the patent: **10.03.2021**
(21) Application number: 15810558.5
(22) Date of filing: 09.06.2015
(51) Int. Cl.: E03D 11/02, G01N 33/50, A61B 10/00, G01N 33/493

(54) **TOILET WITH AUTOMATIC URINE COLLECTING AND TESTING FUNCTION**
TOILETTE MIT AUTOMATISCHER HARNSAMMEL- UND TESTFUNKTION
TOILETTES AVEC FONCTION DE COLLECTE ET DE TEST D'URINE AUTOMATIQUE

(30) Priority: 16.06.2014 CN 201420319010 U
(43) Date of publication of application: 19.04.2017
(73) Proprietor: Zhejiang Deyeetec Medical Technology Co., Ltd., Zhejiang (CN)
(72) Inventor: YE, Zhiquan, Shanghai 201906 (CN)
(74) Representative: Haseltine Lake Kempner LLP
(86) International application number: PCT/CN2015/081036
(87) International publication number: WO 2015/192725

(56) References cited:
- EP-A1- 0 323 901
- CN-A- 101 787 730
- CN-A- 102 041 844
- CN-U- 201 983 948
- CN-U- 201 983 948
- CN-U- 204 023 746
- JP-A- S59 217 844
- JP-A- S63 290 961
- JP-A- 2000 241 409
- JP-B2- 3 304 587
- JP-B2- 3 879 260
- JP-B2- 4 217 880

## Description

### BACKGROUND OF THE INVENTION

### 1. Field of the Invention

The present invention relates to medical instruments field, and more particularly to a toilet with automatic urine collecting and testing function.

### 2. Description of the Prior Art

The prior conventional urine biochemical test is generally conducted by professionals in hospitals and urine collecting should be operated by human so urine testing is very inconvenient.

The prior Chinese patent CN101787790B applied by the present inventor publicizes a toilet with intelligent automatic urine testing function. It can collect and test urine automatically but the structure of collecting urine is more complicated and it doesn't fully comply with the daily habit of people using toilets. Another prior Chinese patent CN102041844B applied by the present inventor publicizes an intelligently testing the structure of toilet urine collecting function. Though it complies with the daily habit of people using toilets, the structure of urine collecting is annular so it's easy to accumulate urine and inconvenient to clean. Besides, there is no urine testing device to match the urine collecting structure. CN 201 983 948 U describes a semi-annular urine collecting tank for fixing to a toilet bowl, with a urine collecting cup connected to the urine collecting tank and a urine collecting tube or catheter provided in the collecting cup and used for connection with a suction device JP 3 304587 B2 discloses a toilet with automatic urine collecting and testing function according to the preamble of claim 1.

### 3. BRIEF SUMMARY OF THE INVENTION

In order to overcome the technical problems, the present invention provides a toilet with automatic urine collecting and testing function.

In order to solve above problems, the present invention provides a toilet with automatic urine collecting and testing function, comprising a toilet body and a urine testing device, characterized in that the toilet body comprises a toilet bowl, the inner wall of the toilet bowl is provided with a section with a urine collecting groove thereon, the length of the urine collecting groove is one half to one fifth of the perimeter of the inner wall of the toilet bowl, one lower end of the urine collecting groove is provided with a urine collecting opening, the opening is provided with a valve device therein, a downward extending urine collecting tube is disposed under the urine collecting opening, the urine collecting tube is connected with urine collecting cup of urine testing device.

As a preferred embodiment, the present urine testing device comprises a urine dispensing device, the urine dispensing device comprising a urine dispensing metering pump, a urine dispensing slide, a drive screw and a drive motor, the drive motor configured to drive the screw so the screw drives the urine dispensing slide to slide;
a testing liquid dispensing device, the testing liquid dispensing device comprising a plurality of testing liquid loading devices, a plurality of testing liquid delivery metering pump and a testing liquid dispensing pipe, the testing liquid dispensing pipe being provided with a plurality of testing liquid dispensing holes, one testing liquid loading device connecting one testing liquid delivery metering pump and one testing liquid dispensing hole;
a reaction device, the reaction device comprising a testing cup and a testing cup bracket, the testing cup bracket and the urine collecting cup being installed on the same rotation axle;
a cleaning device, the cleaning device comprising a cleaning pump, a sewage pump, a cleaning rotating motor and a cleaning pipe, the cleaning pipe is provided with a plurality of cleaning nozzle holes;
the urine dispensing slide and the testing liquid dispensing pipe being just provided above the testing cup bracket, the cleaning pipe being just provided under the testing cup bracket, the cleaning rotating motor configured to drive the rotation axle to rotate the testing cup bracket and the urine collecting cup, when the testing is completed, the testing cup bracket and the urine collecting cup will be cleaned by the beneath cleaning nozzle hole.

The present invention has the advantages that 1) the urine collecting groove of the device is installed reasonably and is convenient to collect urine, does not cause accumulation of urine and is easy to clean; 2) the device and its assorted urine testing device are connected so it realizes automatic urine collecting and testing.

### BRIEF DESCRIPTION OF THE DRAWINGS

FIG. 1 is the top view of the toilet with automatic urine collecting and testing function.
FIG. 2 is the top view of another embodiment
FIG. 3 is the enlarged top view of the urine testing device.
FIG. 4 is the enlarged front view of the urine testing device.

### Reference numerals:

1-toilet body; 11-toilet bowl; 12a, 12b-urine collecting groove; 13-urine collecting open; 2-urine testing device; 21-urine collecting cup; 22-urine dispensing device; 221-urine dispensing metering pump; 222-urine dispensing slick; 223-drive screw; 224-drive motor; 23-testing liquid dispensing device; 231 -testing liquid loading device; 232-testing liquid delivery metering pump; 233-testing liquid dispensing pipe; 234-testing liquid dispensing hole; 24-reaction device; 241-testing cup; 242-testing cup bracket; 25-cleaning device; 251-cleaning pump; 252-sewage pump; 253-cleaning rotating motor; 254-rotation axle; 255-cleaning pipe; 256-cleaning nozzle hole

### DETAILED DESCRIPTION OF THE PREFERRED EMBODIMENTS

The following text will further describe the present invention with reference to the accompanying drawings. The embodiment is only to explain the present invention but not to limit the technical solution.

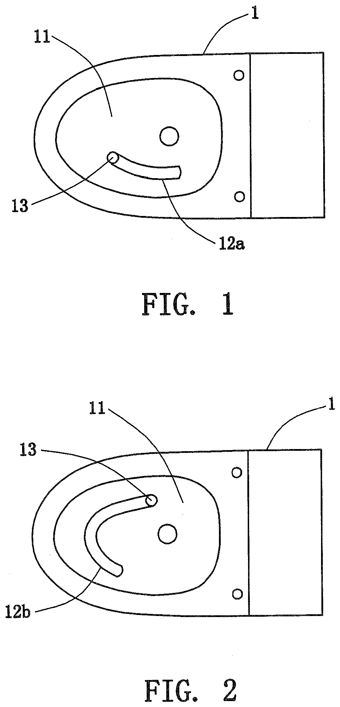

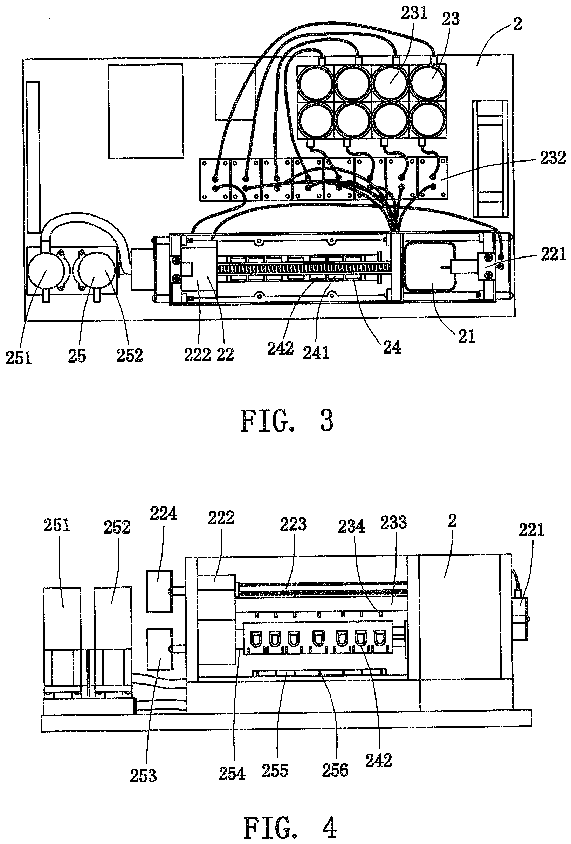

Please refer to FIG. 1 and FIG. 3, a toilet with automatic urine collecting and testing function comprises a toilet body 1 and a urine testing device 2; the toilet body 1 comprises a toilet bowl 11; the inner wall of the toilet bowl 11 is provided with a section with a urine collecting groove 12a thereon, the length of the urine collecting groove 12a is one half to one fifth of the perimeter of the inner wall of the toilet bowl. FIG. 1 is an embodiment of urine collecting groove 12a, the length of the urine collecting groove is one fifth of the perimeter of the inner wall of the toilet bowl. FIG.2 is another embodiment of urine collecting groove, the length of the urine collecting groove is one half of the perimeter of the inner wall of the toilet bowl. The urine collecting groove can be provided on the position shown in FIG. 2 and can be provided on the other half of the semi-circle of the inner wall of the toilet bowl. One lower end of the urine collecting groove is provided with the urine collecting opening. When urine passes through the urine collecting groove, it will accumulate into the urine collecting groove and outflow through the lower urine collecting opening. Chinese patent CN102041844B publicizes an intelligently testing the structure of toilet urine collecting function. Though it complies with the daily habit of people using toilets, the structure of urine collecting is annular , surrounding around the toilet bowl with installment in 360 degree so it's easy to accumulate urine and inconvenient to clean. In fact, after long-term usage, there is no need to set the length of the urine collecting groove as a circle. Only randomly choosing one section can realize the urine collecting function and is not easy to cause accumulation of urine and easy to clean. One lower end of the urine collecting groove is provided urine collecting opening 13, the urine collecting opening 13 is provided with a valve device therein (not shown in the figure), a downward extending urine collecting tube (not shown in the figure) is disposed under the urine collecting opening 13, the urine collecting tube is connected with urine collecting cup 21 of urine testing device 2.

Please refer to FIG. 3 and FIG. 4, urine testing device 2 comprises a urine collecting cup 21 and
a urine dispensing device 22, the urine dispensing device 22 comprising a urine dispensing metering pump 221, a urine dispensing slide 222, a drive screw 223 and a drive motor 224. The drive motor 224 drives the screw 223 so the screw 223 drives the urine dispensing slide 222 to slide;
a testing liquid dispensing device 23, the testing liquid dispensing device 23 comprising a plurality of testing liquid loading devices 231, a plurality of testing liquid delivery metering pumps 232 and a testing liquid dispensing pipe 233, the testing liquid dispensing pipe 233 being provided with a plurality of testing liquid dispensing holes 234, one testing liquid loading device 231 connecting one testing liquid delivery metering pump 232 and one testing liquid dispensing hole 233;
a reaction device 24, the reaction device 24 comprising a testing cup 241 and a testing cup bracket 242. The testing cup bracket 242 and the urine collecting cup 21 are installed on the same rotation axle 254;
a cleaning device 25, the cleaning device 25 comprising a cleaning pump 251, a sewage pump 252, a cleaning rotating motor 253 and a cleaning pipe 255. The cleaning pipe 255 is provided with a plurality of cleaning nozzle holes 256.

The urine dispensing metering pump 221 sucks quantitative urine from the urine collecting cup 21 and drops it in turn into the testing cup 241 through gliding of the urine dispensing slide 222. The urine dispensing slide 222 and the testing liquid dispensing pipe 233 are just provided above the testing cup bracket 242. The cleaning pipe 255 is just provided under the testing cup bracket 242. The cleaning rotating motor 253 drives the rotation axle 254 to rotate the testing cup bracket 242 and the urine collecting cup 21. When the testing is completed, the testing cup bracket 242 and the urine collecting cup 21 will be cleaned by the beneath cleaning nozzle hole 256.

FIG. 4 is the front view of the rotating testing cup bracket 242 of the urine collecting and testing device 2. The testing cup bracket 242 and the urine collecting cup 21 can rotate in 360 degree. When cleaning, rotate the rotation axle 254 and keep the testing cup bracket 242 and the urine collecting cup 21 downwards facing the cleaning nozzle hole 256. Sewage will be drained through sewage pump 252.

The urine testing device has compact structure, reasonable layout and small size. When using in a toilet with urine collecting function, it can realize complete automation including automatic urine collecting, dispensing, testing and cleaning.

The above description is just the preferred embodiment of the present invention. Any person who skilled in the art may deform and modify the embodiment of the present invention within the scope of the appended claims.

## Claims

1. A toilet with automatic urine collecting and testing function comprising a toilet body (1) and a urine testing device (2), wherein the toilet body (1) comprises a toilet bowl (11); **characterized in that** the inner wall of the toilet bowl (11) is provided with a section with a urine collecting groove (12a, 12b) thereon; the length of the urine collecting groove (12a, 12b) is one half to one fifth of the perimeter of the inner wall of the toilet bowl (11); one lower end of the urine collecting groove (12a, 12b) is provided with a urine collecting opening (13); the opening (13) is provided with a valve device therein, a downward extending urine collecting tube is disposed under the urine collecting opening (13), the urine collecting tube is connected with urine collecting cup (21) of urine testing device (2).

2. The toilet with automatic urine collecting and testing function as claimed in Claim 1, **characterized in that** the urine testing device (2) comprises a urine dispensing device (22), the urine dispensing device (22) comprising a urine dispensing metering pump (221), a urine dispensing slide (222), a drive screw (223) and a drive motor (224), the drive motor (224) is configured to drive the screw (223) so the screw (223) drives the urine dispensing slide (222) to slide;
a testing liquid dispensing device (23), the testing liquid dispensing device (230 comprising a plurality of testing liquid loading devices (231), a plurality of testing liquid delivery metering pumps (232) and a testing liquid dispensing pipe (233), the testing liquid dispensing pipe (233) being provided with a plurality of testing liquid dispensing holes (234), one testing liquid loading device (231) connecting one testing liquid delivery metering pump (232) and one testing liquid dispensing hole (234);
a reaction device (24), the reaction device (24) comprising a testing cup (241) and a testing cup bracket (242), the testing cup bracket (242) and the urine collecting cup (21) being installed on the same rotation axle (254);
a cleaning device (25), the cleaning device (25) comprising a cleaning pump (251), a sewage pump (252), a cleaning rotating motor (253) and a cleaning pipe (255), the cleaning pipe (255) being provided with a plurality of cleaning nozzle holes (256);
the urine dispensing slide (222) and the testing liquid dispensing pipe (233) being provided above the testing cup bracket (242), the cleaning pipe (255) being provided under the testing cup bracket (242), the cleaning rotating motor (253) being configured to drive the rotation axle (254) to rotate the testing cup bracket (242) and the urine collecting cup (21), when the testing is completed, the testing cup bracket (242) and the urine collecting cup (21) will be cleaned by the beneath cleaning nozzle hole (256).

## Patentansprüche

1. Toilette mit automatischer Urinsammel- und Testfunktion, umfassend einen Toilettenkörper (1) und eine Urintestvorrichtung (2), wobei der Toilettenkörper (1) eine Toilettenschüssel (11) umfasst; **dadurch gekennzeichnet, dass** die Innenwand der Toilettenschüssel (11) mit einem Abschnitt mit einer Urinsammelnut (12a, 12b) darauf versehen ist; die Länge der Urinsammelnut (12a, 12b) die Hälfte bis ein Fünftel des Umfangs der Innenwand der Toilettenschüssel (11) ist; ein unteres Ende der Urinsammelnut (12a, 12b) mit einer Urinsammelöffnung (13) versehen ist; die Öffnung (13) mit einer Ventilvorrichtung darin versehen ist, ein sich nach unten erstreckendes Urinsammelrohr unter der Urinsammeiöffnung (13) angeordnet ist, das Urinsammelrohr mit einem Urinsammelbecher (21) der Urintestvorrichtung (2) verbunden ist.

2. Toilette mit automatischer Urinsammel- und Testfunktion gemäß Anspruch 1, **dadurch gekennzeichnet, dass** die Urintestvorrichtung (2) eine Urinausgabevorrichtung (22) umfasst, die Urinausgabevorrichtung (22) umfassend eine Urindosierpumpe (221), einen Urinausgabeschieber (222), eine Antriebsschraube (223) und einen Antriebsmotor (224), wobei der Antriebsmotor (224) zum Antreiben der Schraube (223) konfiguriert ist, sodass die Schraube (223) den Urinausgabeschieber (222) antreibt, um zu gleiten;
eine Testflüssigkeit-Ausgabevorrichtung (23), wobei die Testflüssigkeit-Ausgabevorrichtung (230) eine Vielzahl von Testflüssigkeit-Füllvorrichtungen (231), eine Vielzahl von Testflüssigkeit-Ausgabedosierpumpen (232) und ein Testflüssigkeit-Ausgaberohr (233) umfasst, wobei das Testflüssigkeit-Ausgaberohr (233) mit einer Vielzahl von Testflüssigkeit-Ausgabelöchern (234) versehen ist, wobei eine Testflüssigkeit-Füllvorrichtung (231) eine Testflüssigkeit-Ausgabedosierpumpe (232) und ein Testflüssigkeit-Ausgabeloch (234) verbindet; eine Reaktionsvorrichtung (24), die Reaktionsvorrichtung (24) umfassend einen Testbecher (241) und eine Testbecherhalterung (242), wobei die Testbecherhalterung (242) und der Urinsammelbecher (21) auf derselben Drehachse (254) installiert sind;
eine Reinigungsvorrichtung (25), die Reinigungsvorrichtung (25) umfassend eine Reinigungspumpe (251), eine Abwasserpumpe (252), einen Reinigungsdrehmotor (253) und ein Reinigungsrohr (255), wobei das Reinigungsrohr (255) mit einer Vielzahl von Reinigungsdüsenlöchern (256) versehen ist;
der Urinausgabeschieber (222) und das Testflüssigkeit-Ausgaberohr (233) oberhalb der Testbecherhalterung (242) bereitgestellt sind, das Reinigungsrohr (255) unter der Testbecherhalterung (242) bereitgestellt ist, der Reinigungsdrehmotor (253) konfiguriert ist, um die Drehachse (254) anzutreiben, um die Testbecherhalterung (242) und den Urinsammelbecher (21) zu drehen, wobei, wenn der Test abgeschlossen ist, die Testbecherhalterung (242) und der Urinsammelbecher (21) durch das untere Reinigungsdüsenloch (256) gereinigt werden.

## Revendications

1. Toilettes avec une fonction automatique de collecte et de test d'urine comprenant un corps de toilettes (1) et un dispositif de test d'urine (2), dans lesquelles le corps de toilettes (1) comprend une cuvette de toilettes (11) ; **caractérisé en ce que** la paroi intérieure de la cuvette de toilettes (11) est munie d'une section avec une rainure de collecte d'urine (12a, 12b) sur celle-ci ; la longueur de la rainure de collecte d'urine (12a, 12b) est d'une moitié à un cinquième du périmètre de la paroi intérieure de la cuvette de toilettes (11) ; une extrémité inférieure de la rainure de collecte d'urine (12a, 12b) est munie d'une ouverture de collecte d'urine (13) ; l'ouverture (13) est munie d'un dispositif de soupape dans celle-ci, un tube de collecte d'urine s'étendant vers le bas est disposé sous l'ouverture de collecte d'urine (13), le tube de collecte d'urine est relié à la tasse de collecte d'urine (21) du dispositif de test d'urine (2).

2. Toilettes avec une fonction automatique de collecte et de test d'urine selon la revendication 1, **caractérisée en ce que** le dispositif de test d'urine (2) comprend un dispositif de distribution d'urine (22), le dispositif de distribution d'urine (22) comprenant une pompe doseuse de distribution d'urine (221), une lame de distribution d'urine (222), une vis d'entraînement (223) et un moteur d'entraînement (224), le moteur d'entraînement (224) est configuré pour entraîner la vis (223) de sorte que la vis (223) entraîne la lame de distribution d'urine (222) à coulisser ;
un dispositif de distribution de liquide de test (23), le dispositif de distribution de liquide de test (230) comprenant une pluralité de dispositifs de chargement de liquide de test (231), une pluralité de pompes doseuses de distribution de liquide de test (232) et un tuyau de distribution de liquide de test (233), le tuyau de distribution de liquide de test (233) étant pourvu d'une pluralité de trous de distribution de liquide de test (234), un dispositif de chargement de liquide de test (231) reliant une pompe doseuse de distribution de liquide de test (232) et un trou de distribution de liquide de test (234) ;
un dispositif de réaction (24), le dispositif de réaction (24) comprenant une tasse de test (241) et un support de la tasse de test (242), le support de la tasse de test (242) et la tasse de collecte d'urine (21) étant installés sur le même axe de rotation (254) ;
un dispositif de nettoyage (25), le dispositif de nettoyage (25) comprenant une pompe de nettoyage (251), une pompe d'eaux usées (252), un moteur rotatif de nettoyage (253) et un tuyau de nettoyage (255), le tuyau de nettoyage (255) étant muni d'une pluralité de trous de buses de nettoyage (256) ;
la lame de distribution d'urine (222) et le tuyau de distribution de liquide de test (233) étant prévus au-dessus du support de la tasse de test (242), le tuyau de nettoyage (255) étant prévu sous le support de la tasse de test (242), le moteur rotatif de nettoyage (253) étant configuré pour entraîner l'axe de rotation (254) afin de faire tourner le support de la tasse de test (242) et la tasse de collecte d'urine (21), lorsque le test est terminé, le support de la tasse de test (242) et la tasse de collecte d'urine (21) seront nettoyés par le trou de la buse de nettoyage situé en dessous (256).
